(19) Europäisches Patentamt / European Patent Office / Office européen des brevets

(11) **EP 3 005 945 A1**

(12) **EUROPEAN PATENT APPLICATION**
published in accordance with Art. 153(4) EPC

(43) Date of publication:
**13.04.2016 Bulletin 2016/15**

(51) Int Cl.:
**A61B 8/00** *(2006.01)*

(21) Application number: **14804957.0**

(22) Date of filing: **27.05.2014**

(86) International application number:
**PCT/JP2014/064559**

(87) International publication number:
**WO 2014/192954 (04.12.2014 Gazette 2014/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **29.05.2013 JP 2013113284**

(71) Applicant: **OLYMPUS CORPORATION**
**Shibuya-ku**
**Tokyo 151-0072 (JP)**

(72) Inventor: **NOGUCHI, Hiromasa**
**Tokyo 151-0072 (JP)**

(74) Representative: **Gunzelmann, Rainer**
**Wuesthoff & Wuesthoff**
**Patentanwälte PartG mbB**
**Schweigerstraße 2**
**81541 München (DE)**

(54) **ULTRASONIC OBSERVATION DEVICE, OPERATION METHOD FOR ULTRASONIC OBSERVATION DEVICE, AND OPERATION PROGRAM FOR ULTRASONIC OBSERVATION DEVICE**

(57) An ultrasonic observation apparatus includes an ultrasonic probe for transmitting an ultrasonic signal to a specimen and receiving an ultrasonic wave reflected from the specimen, a frequency analysis unit for calculating a frequency spectrum by analyzing a frequency of the ultrasonic wave received by the ultrasonic probe (2), a feature-data extraction unit for extracting at least one piece of feature data from the frequency spectrum by approximating the frequency spectrum calculated by the frequency analysis unit, and a feature-data image data generation unit for generating feature-data image data that displays information corresponding to the feature data in accordance with one of a plurality of display methods depending on a relationship between the feature data extracted by the feature-data extraction unit and a threshold in the feature data, the threshold being constant regardless of a value of a display parameter which the image data has.

FIG.1

EP 3 005 945 A1

**Description**

Field

The present invention relates to an ultrasonic observation apparatus for observing a tissue of a specimen by using ultrasonic waves, a method for operating the ultrasonic observation apparatus, and a program for operating the ultrasonic observation apparatus.

Background

Conventionally, a technique for imaging feature data of a frequency spectrum of a received ultrasonic signal is known as a technique using ultrasonic waves for observing a tissue characteristics to be observed, such as a specimen (for example, see Patent Literature 1). According to this technique, after the feature data of the frequency spectrum is extracted as an amount representing the tissue characteristics to be observed, a feature-data image to which visual information corresponding to the feature data is provided is generated and displayed. A user, such as a doctor, diagnoses the tissue characteristics of the specimen by observing the displayed feature-data image.

Citation List

Patent Literature

Patent Literature 1: WO2012/063975

Summary

Technical Problem

However, according to the above-described conventional technique, luminance and color of a pixel that forms the feature-data image vary depending on a value of a display parameter required for imaging, such as gain and contrast, and have poor correlation with a value of the feature data. For this reason, according to the above-described conventional technique, it is sometimes difficult for a user to objectively and precisely diagnose the tissue characteristics to be observed.
The present invention has been made in view of the foregoing and an object of the invention is to provide an ultrasonic observation apparatus, a method for operating the ultrasonic observation apparatus, and a program for operating the ultrasonic observation apparatus that allow a user to objectively and precisely diagnose tissue characteristics to be observed.

Solution to Problem

To solve the problem explained above and to achieve the object, an ultrasonic observation apparatus according to the invention includes: an ultrasonic probe configured to transmit an ultrasonic signal to a specimen and to receive an ultrasonic wave reflected from the specimen; a frequency analysis unit configured to analyze a frequency of the ultrasonic wave received by the ultrasonic probe to calculate a frequency spectrum; a feature-data extraction unit configured to approximate the frequency spectrum calculated by the frequency analysis unit to extract at least one piece of feature data from the frequency spectrum; and a feature-data image data generation unit configured to generate feature-data image data for displaying information corresponding to the feature data in accordance with one of a plurality of display methods, depending on a relationship between the feature data extracted by the feature-data extraction unit and a threshold in the feature data, the threshold being constant regardless of a value of a display parameter of image data.
In the above-described invention, the ultrasonic observation apparatus according to the invention further includes: a threshold information storage unit configured to associate a value of the feature data including the threshold with the plurality of display methods, and to store the value of the feature data including the threshold and the plurality of display methods; and a display method selector configured to select a display method of the information corresponding to the feature data extracted by the feature-data extraction unit from among the plurality of display methods stored in the threshold information storage unit, and to cause the feature-data image data generation unit to generate the feature-data image data in accordance with the selected display method.
According to the ultrasonic observation apparatus of the invention, in the above-described invention, the plurality of display methods are classified into color display in which hue varies depending on the value of the feature data, and gray scale display in which hue is constant regardless of the value of the feature data. The display method selector is configured to select one of the color display and the gray scale display in accordance with a magnitude relationship between the feature data and the threshold.

According to the ultrasonic observation apparatus of the invention, in the above-described invention, the ultrasonic probe can be selected from a plurality of ultrasonic probes different in types from one another. The threshold information storage unit stores the threshold according to types of the specimen and the ultrasonic probe. The feature-data image data generation unit is configured to generate the feature-data image data based on the threshold according to the types of the specimen and the ultrasonic probe.

According to the ultrasonic observation apparatus of the invention, in the above-described invention, the feature-data extraction unit includes: an approximation unit configured to perform approximation processing on the frequency spectrum calculated by the frequency analysis unit to calculate an approximate expression of the frequency spectrum; and an attenuation correction unit configured to perform attenuation correction processing for reducing contribution of attenuation that occurs in accordance with a receiving depth and frequency of the ultrasonic wave, on the approximate expression calculated by the approximation unit to extract the feature data of the frequency spectrum.

According to the ultrasonic observation apparatus of the invention, in the above-described invention, the feature-data extraction unit includes: an attenuation correction unit configured to perform attenuation correction processing for reducing contribution of attenuation that occurs in accordance with a receiving depth and frequency of the ultrasonic wave, on the frequency spectrum calculated by the frequency analysis unit when the ultrasonic wave propagates; and an approximation unit configured to perform approximation processing on the frequency spectrum corrected by the attenuation correction unit to extract the feature data of the frequency spectrum.

According to the ultrasonic observation apparatus of the invention, in the above-described invention, the approximation unit is configured to approximate the frequency spectrum as an approximation target by a polynomial through regression analysis.

According to the ultrasonic observation apparatus of the invention, in the above-described invention, the approximation unit is configured to approximate the frequency spectrum as the approximation target by a linear expression, and to extract, as the feature data, at least one of a slope of the linear expression, an intercept of the linear expression, and intensity that is defined by using the slope, the intercept, and a specific frequency included in a frequency band of the frequency spectrum.

A method for operating an ultrasonic observation apparatus according to the invention is a method for operating an ultrasonic observation apparatus that transmits an ultrasonic signal to a specimen and receives an ultrasonic wave reflected from the specimen. The method includes: a frequency analysis step of analyzing, by a frequency analysis unit, a frequency of the ultrasonic wave to calculate a frequency spectrum; a feature-data extraction step of approximating, by a feature-data extraction unit, the frequency spectrum to extract at least one piece of feature data from the frequency spectrum; and a feature-data image data generation step of generating, by a feature-data image data generation unit, feature-data image data for displaying information corresponding to the feature data in accordance with one of a plurality of display methods, depending on a relationship between the feature data extracted in the feature-data extraction step and a threshold in the feature data, the threshold being constant regardless of a value of a display parameter of image data.

A program for operating an ultrasonic observation apparatus according to the invention causes the ultrasonic observation apparatus that transmits an ultrasonic signal to a specimen and receives an ultrasonic wave reflected from the specimen to execute: a frequency analysis step of analyzing, by a frequency analysis unit, a frequency of the ultrasonic wave to calculate a frequency spectrum; a feature-data extraction step of approximating, by a feature-data extraction unit, the frequency spectrum to extract at least one piece of feature data from the frequency spectrum; and a feature-data image data generation step of generating, by a feature-data image data generation unit, feature-data image data for displaying information corresponding to the feature data in accordance with one of a plurality of display methods, depending on a relationship between the feature data extracted in the feature-data extraction step and a threshold in the feature data, the threshold being constant regardless of a value of a display parameter of image data.

Advantageous Effects of Invention

The present invention allows a user to objectively and precisely diagnose the tissue characteristics to be observed.

Brief Description of Drawings

FIG. 1 is a block diagram illustrating a configuration of an ultrasonic observation apparatus according to one embodiment of the present invention.

FIG. 2 is a diagram illustrating a relationship between a receiving depth and an amplification factor in amplification processing performed by a signal amplification unit of the ultrasonic observation apparatus according to one embodiment of the present invention.

FIG. 3 is a diagram illustrating the relationship between the receiving depth and the amplification factor in amplification processing performed by an amplification correction unit of the ultrasonic observation apparatus according to one embodiment of the present invention.

FIG. 4 is a diagram illustrating an example of a frequency spectrum calculated by a frequency analysis unit of the ultrasonic observation apparatus according to one embodiment of the present invention.

FIG. 5 is a diagram illustrating straight lines corresponding to feature data corrected by an attenuation correction unit of the ultrasonic observation apparatus according to one embodiment of the present invention.

FIG. 6 is a diagram illustrating a display example of a B-mode image corresponding to B-mode image data generated by a B-mode image generation unit of the ultrasonic observation apparatus according to one embodiment of the present invention.

FIG. 7 is a diagram illustrating a relationship between the feature data and a plurality of display methods when a feature-data image data generation unit of the ultrasonic observation apparatus according to one embodiment of the present invention generates feature-data image data.

FIG. 8 is a diagram schematically illustrating an image illustrated in FIG. 7 in black and white.

FIG. 9 is a diagram illustrating an example of threshold information stored in a threshold information storage unit of the ultrasonic observation apparatus according to one embodiment of the present invention.

FIG. 10 is a flow chart illustrating an overview of processing of the ultrasonic observation apparatus according to one embodiment of the present invention.

FIG. 11 is a flow chart illustrating an overview of processing performed by a frequency analysis unit of the ultrasonic observation apparatus according to one embodiment of the present invention.

FIG. 12 is a diagram schematically illustrating data arrangement of one sound ray.

FIG. 13 is a diagram schematically illustrating an example of a feature-data image displayed by a display unit of the ultrasonic observation apparatus according to one embodiment of the present invention.

FIG. 14 is a diagram schematically illustrating another method of setting hue when the feature-data image data generation unit of the ultrasonic observation apparatus according to one embodiment of the present invention generates the feature-data image data.

FIG. 15 is a diagram schematically illustrating the image illustrated in FIG. 14 in black and white.

FIG. 16 is a diagram illustrating an example of a case where the display unit of the ultrasonic observation apparatus according to another embodiment of the present invention superimposes the B-mode image on the feature-data image and displays the B-mode image and the feature-data image.

FIG. 17 is a diagram schematically illustrating the image illustrated in FIG. 16 in black and white.

FIG. 18 is a diagram schematically illustrating an overview of attenuation correction processing performed by an attenuation correction unit of the ultrasonic observation apparatus according to another embodiment of the present invention.

Description of Embodiments

Modes for carrying out the invention (hereinafter referred to as "embodiments") will be described below with reference to the accompanying drawings.

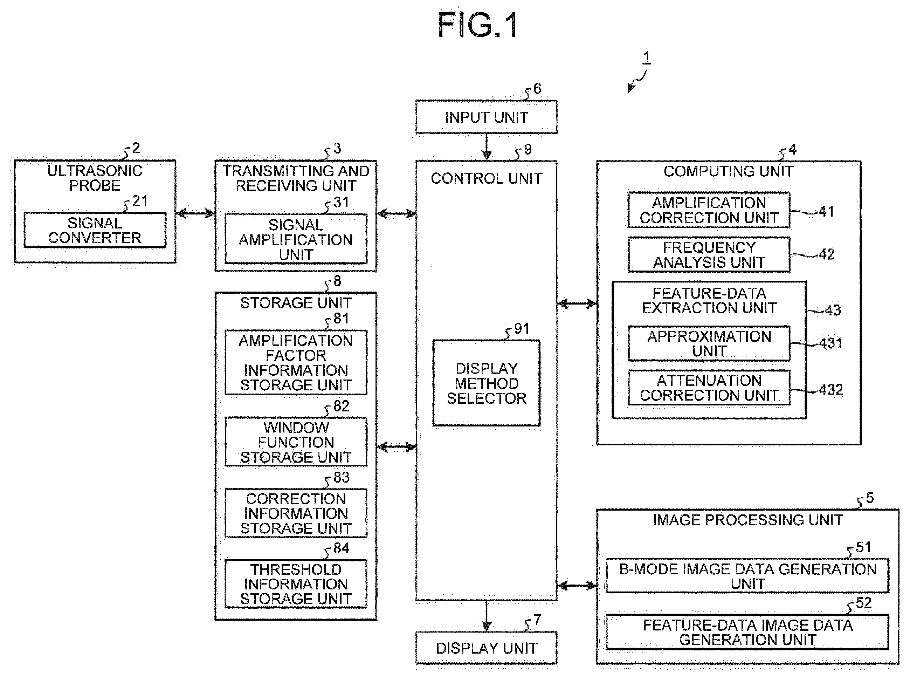

FIG. 1 is a block diagram illustrating a configuration of an ultrasonic observation apparatus according to one embodiment of the present invention. An ultrasonic observation apparatus 1 illustrated in FIG. 1 is an apparatus for observing a specimen to be diagnosed by using ultrasonic waves. The ultrasonic observation apparatus 1 includes an ultrasonic probe 2 that outputs an ultrasonic pulse to the outside and receives an externally reflected ultrasonic echo, a transmitting and receiving unit 3 that transmits and receives an electric signal to and from the ultrasonic probe 2, a computing unit 4 that performs specified computations on an electric echo signal obtained by converting the ultrasonic echo, an image processing unit 5 that generates image data corresponding to the electric echo signal, an input unit 6 that is implemented by using an interface, such as a keyboard, a mouse, or a touch panel, and that receives input of various pieces of information, a display unit 7 that is implemented by using a display panel including a liquid crystal or organic EL and that displays various pieces of information including an image generated by the image processing unit 5, a storage unit 8 that stores various pieces of information required for ultrasonic observation, and a control unit 9 that performs operation control of the ultrasonic observation apparatus 1. The ultrasonic observation apparatus 1 includes a scope that has the ultrasonic probe 2 provided at a distal end, and a processor to which a proximal end of the scope is detachably connected, the processor being provided with the above-described units other than the ultrasonic probe 2.

The ultrasonic probe 2 has a signal converter 21 that converts an electric pulse signal received from the transmitting and receiving unit 3 into an ultrasonic pulse (acoustic pulse signal), and that converts the ultrasonic echo reflected from the external specimen into the electric echo signal. The ultrasonic probe 2 may mechanically scan an ultrasonic transducer, or may electronically scan the plurality of ultrasonic transducers. According to the embodiment, it is possible to select and use, as the ultrasonic probe 2, one of a plurality of types of ultrasonic probes 2 different from one another.

The transmitting and receiving unit 3 is electrically connected to the ultrasonic probe 2, transmits the pulse signal to the ultrasonic probe 2, and receives the electric echo signal that is a reception signal from the ultrasonic probe 2. Specifically, the transmitting and receiving unit 3 generates the pulse signal based on a previously set waveform and transmitting timing, and transmits the generated pulse signal to the ultrasonic probe 2.

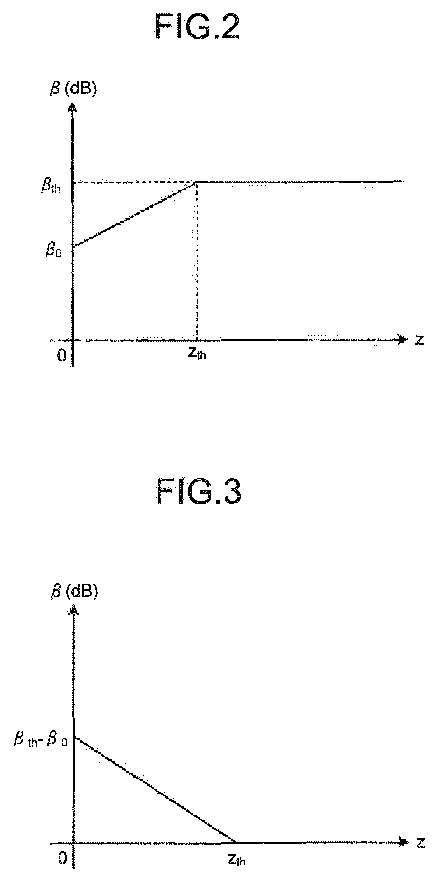

The transmitting and receiving unit 3 has a signal amplification unit 31 that amplifies the echo signal. Specifically, the signal amplification unit 31 performs STC correction to amplify the echo signal with higher amplification factor as a receiving depth of the echo signal increases. FIG. 2 is a diagram illustrating a relationship between the receiving depth and the amplification factor in amplification processing performed by the signal amplification unit 31. The receiving depth z illustrated in FIG. 2 is an amount calculated based on an elapsed time from a time point at which reception of the ultrasonic wave is started. As illustrated in FIG. 2, when the receiving depth z is smaller than a threshold $z_{th}$, the amplification factor P (dB) increases linearly from $\beta_0$ to $\beta_{th}$ ($> \beta_0$) as the receiving depth z increases. In addition, when the receiving depth z is equal to or greater than the threshold $z_{th}$, the amplification factor $\beta$ takes a constant value $\beta_{th}$. A value of the threshold $z_{th}$ is a value at which the ultrasonic signal received from the specimen is almost attenuated and noise is dominant. More generally, the amplification factor $\beta$ may monotonically increase as the receiving depth z increases when the receiving depth z is less than the threshold $z_{th}$.

After performing processing, such as filtering, on the echo signal amplified by the signal amplification unit 31, the transmitting and receiving unit 3 performs analog-to-digital conversion on the processed signal to generate and output a time-domain digital RF signal. Here, when the ultrasonic probe 2 electronically scans a plurality of ultrasonic transducers, the transmitting and receiving unit 3 has a multi-channel circuit that supports a plurality of ultrasonic transducers for beam synthesis.

The computing unit 4 has an amplification correction unit 41 that performs amplification correction to make the amplification factor constant with respect to the digital RF signal that is output from the transmitting and receiving unit 3 regardless of the receiving depth, a frequency analysis unit 42 that calculates a frequency spectrum by applying fast Fourier transform (FFT) to the digital RF signal that undergoes amplification correction to perform frequency analysis, and a feature-data extraction unit 43 that extracts feature data of the specimen by performing approximation processing on the frequency spectrum at each point calculated by the frequency analysis unit 42 based on regression analysis, and attenuation correction processing for reducing contribution of attenuation that occurs depending on the receiving depth and a frequency of an ultrasonic wave when the ultrasonic wave propagates.

FIG. 3 is a diagram illustrating the relationship between the receiving depth and the amplification factor in amplification processing performed by the amplification correction unit 41. As illustrated in FIG. 3, the amplification factor P (dB) in amplification processing that is performed by the amplification correction unit 41 takes a maximum value $\beta_{th}$ - $\beta_0$ when the receiving depth z is zero, decreases linearly while the receiving depth z increases from zero to the threshold $z_{th}$, and is zero when the receiving depth z is equal to or greater than the threshold $z_{th}$. When the amplification correction unit 41 performs amplification correction on the digital RF signal with the amplification factor determined in this way, an influence of the STC correction in the signal amplification unit 31 can be offset, and a signal having the constant amplification factor $\beta_{th}$ can be output. Note that, of course, the relationship between the receiving depth z and the amplification factor $\beta$ performed by the amplification correction unit 41 changes in accordance with the relationship between the receiving depth and the amplification factor in the signal amplification unit 31.

A reason to perform such amplification correction will be described. The STC correction is a correction for amplifying amplitude of an analog signal waveform uniformly over an entire frequency band. Accordingly, while a sufficient effect can be obtained by performing the STC correction when generating a B-mode image that uses amplitude of an ultrasonic wave, an influence of attenuation following propagation of the ultrasonic wave cannot be accurately eliminated when calculating the frequency spectrum of the ultrasonic wave. In order to solve this situation, while a reception signal that undergoes the STC correction is output when generating the B-mode image, new transmission different from transmission for generating the B-mode image may be performed to output a reception signal that has not undergone the STC correction when generating an image based on the frequency spectrum. In this case, however, there is a problem that a frame rate of image data generated based on the reception signal may decline. Therefore, in the embodiment, the amplification correction unit 41 performs correction of the amplification factor in order to eliminate the influence of the STC correction once on the signal that undergoes the STC correction for the B-mode image while maintaining the frame rate of the generated image data.

For each sound ray (line data), the frequency analysis unit 42 calculates the frequency spectrum at a plurality of points (data position) on the sound ray by applying fast Fourier transform to the FFT data group made of a specified data amount. A result calculated by the frequency analysis unit 42 is obtained as a complex number and is stored in the storage unit 8.

Generally, the frequency spectrum shows tendencies that differ depending on the tissue characteristics of a specimen. This is because the frequency spectrum is correlated with a size, density, acoustic impedance, and the like of the specimen that serves as a scatterer for scattering an ultrasonic wave. Note that, in the embodiment, examples of "tissue characteristics" include one of a cancer, an endocrine tumor, a mucinous tumor, a normal tissue, and a vascular channel.

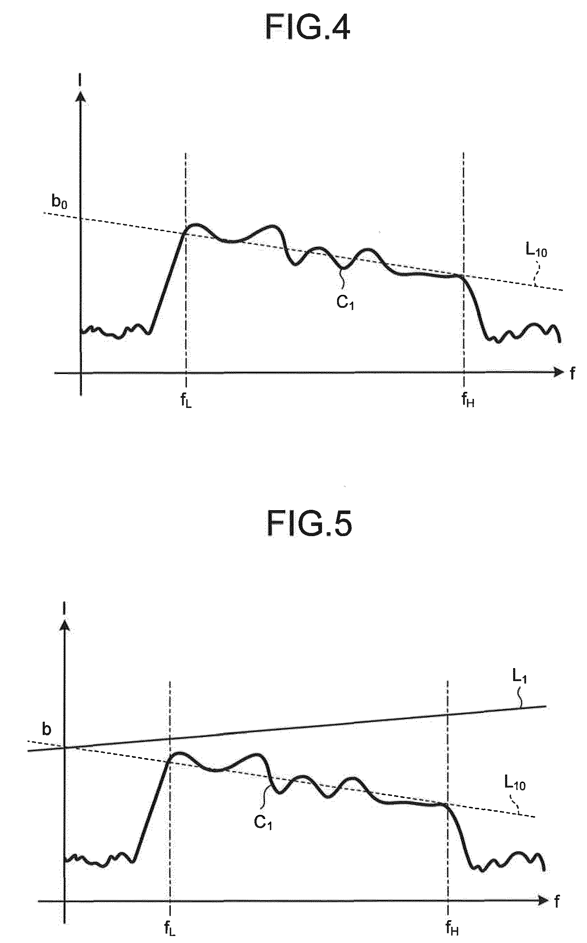

FIG. 4 is a diagram illustrating an example of the frequency spectrum calculated by the frequency analysis unit 42. Specifically, FIG. 4 illustrates the spectrum of intensity I (f, z) when the frequency spectrum obtained by performing fast Fourier transform on the FFT data group is represented by intensity I (f, z) and phase $\phi$ (f, z), where the frequency f and receiving depth are each a function of z. The "intensity" mentioned here refers to one of parameters, such as voltage, electric power, sound pressure, and acoustic energy. In FIG. 4, the horizontal axis f is frequency, the vertical axis I is intensity, and the receiving depth z is constant. In the frequency spectrum curve $C_1$ illustrated in FIG. 4, a lower limit frequency $f_L$ and upper limit frequency $f_H$ of the frequency spectrum are parameters determined based on a frequency band of the ultrasonic probe 2, a frequency band of the pulse signal transmitted by the transmitting and receiving unit 3, and the like. For example, $f_L$ = 3 MHz, and $f_H$ = 10 MHz. In the embodiment, the curve and straight line are formed of a set of discrete points.

The feature-data extraction unit 43 has an approximation unit 431 that calculates an approximate expression of the frequency spectrum calculated by the frequency analysis unit 42 through regression analysis, and an attenuation correction unit 432 that extracts feature data of the frequency spectrum by applying attenuation correction processing for reducing contribution of attenuation of the ultrasonic wave depending on the receiving depth and frequency of the ultrasonic wave to the approximate expression calculated by the approximation unit 431.

The approximation unit 431 approximates the frequency spectrum with a linear expression (regression line) through regression analysis to extract pre-correction feature data characterizing the approximated linear expression. Specifically, the approximation unit 431 extracts a slope $a_0$ and intercept $b_0$ of the linear expression as the pre-correction feature data. The straight line $L_{10}$ illustrated in FIG. 4 is a straight line corresponding to the linear expression approximated by the approximation unit 431. Note that the approximation unit 431 may calculate intensity (also referred to as mid-band fit) $c_0 = a_0 f_M + b_0$ that is a value on the regression line at a center frequency $f_M = (f_L + f_H)/2$ of a frequency band ($f_L < f < f_H$) as pre-correction feature data other than the slope $a_0$ and intercept $b_0$.

It is considered that, among the three pieces of feature data, the slope $a_0$ is correlated with a size of the ultrasonic scatterer, and that generally the slope has a smaller value as the scatterer is larger. In addition, the intercept $b_0$ is correlated with the size of the scatterer, a difference in the acoustic impedance, number density (concentration) of the scatterer, and the like. Specifically, it is considered that the intercept $b_0$ has a lager value as the scatterer is larger, that the intercept $b_0$ has a lager value as the acoustic impedance is larger, and that the intercept $b_0$ has a larger value as the density (concentration) of the scatterer is larger. The intensity $c_0$ at the center frequency $f_M$ (hereinafter, simply referred to as "intensity") is an indirect parameter derived from the slope $a_0$ and the intercept $b_0$, and gives spectrum intensity at a center in the effective frequency band. Therefore, it is considered that the intensity $c_0$ is correlated to some extent with luminance of the B-mode image in addition to the size of the scatterer, the difference in the acoustic impedance, and the density of the scatterer. Note that an approximate polynomial that is calculated by the feature-data extraction unit 43 is not limited to the linear expression, and a quadratic or higher-order approximate polynomial can also be used.

Correction performed by the attenuation correction unit 432 will be described. Generally, an ultrasonic attenuation amount A (f, z) is represented as follows:

$$A \ (f, \ z) \ = \ 2\alpha z f \qquad\qquad\qquad (1)$$

Here, $\alpha$ is an attenuation factor, z is a receiving depth of an ultrasonic wave, and f is a frequency. As is obvious from Equation (1), the attenuation amount A (f, z) is proportional to the frequency f. A specific value of the attenuation factor $\alpha$ is, when a living body is to be observed, in a range of 0.0 to 1.0 (dB/cm/MHz), and more preferably 0.3 to 0.7 (dB/cm/MHz), and is determined in accordance with a region of the living body. For example, when a pancreas is to be observed, it may be determined that $\alpha$ = 0.6 (dB/cm/MHz). Here, in the embodiment, a configuration can also be employed in which the value of the attenuation factor $\alpha$ can be set or changed by an input from the input unit 6.

The attenuation correction unit 432 extracts the feature data by performing attenuation correction on the pre-correction feature data (slope $a_0$, intercept $b_0$, intensity $c_0$) extracted by the approximation unit 431 as follows:

$$a \ = \ a_0 \ + \ 2\alpha z \qquad\qquad\qquad (2)$$

$$b \ = \ b_0 \qquad\qquad\qquad (3)$$

$$c = c_0 + 2\alpha z f_M \ (= a f_M + b) \tag{4}$$

As is obvious from Equations (2) and (4), the attenuation correction unit 432 performs correction with a larger correction amount as the receiving depth z of the ultrasonic wave increases. In addition, according to Equation (3), correction related to the intercept is identical transformation. This is because the intercept is a frequency component corresponding to the frequency of 0 (Hz) and is not affected by attenuation.

FIG. 5 is a diagram illustrating straight lines corresponding to the feature data corrected by the attenuation correction unit 432. The equation representing the straight line $L_1$ is given by:

$$I = af + b = (a_0 + 2\alpha z) \ f + b_0 \tag{5}$$

As is obvious from Equation (5), the straight line $L_1$ is inclined more than the straight line $L_{10}$ and has the same intercept as the intercept of the straight line $L_{10}$.

The image processing unit 5 has a B-mode image data generation unit 51 that generates B-mode image data from the echo signal, and a feature-data image data generation unit 52 that generates feature-data image data for displaying information corresponding to the feature data extracted by the feature-data extraction unit 43 in accordance with one of a plurality of display methods.

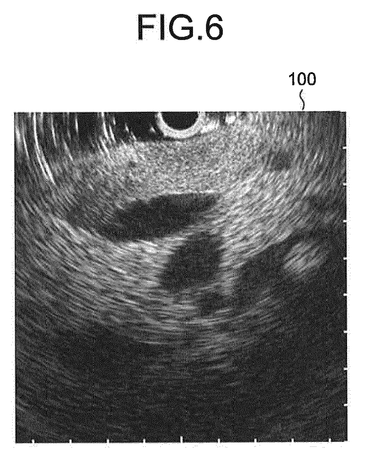

The B-mode image data generation unit 51 performs, on a digital signal, signal processing using a known technique such as a band-pass filter, logarithmic transformation, gain processing, or contrast processing, and performs data decimation according to a data step width determined in accordance with a display range of an image in the display unit 7, thereby generating the B-mode image data. FIG. 6 is a diagram illustrating a display example of the B-mode image corresponding to the B-mode image data generated by the B-mode image data generation unit 51. A B-mode image 100 illustrated in FIG. 6 is a gray scale image in which values of R (red), G (green), and B (blue), which are variables when a RGB color system is employed as a color space, are matched. Note that, when the ultrasonic observation apparatus 1 is specialized for generation of feature-data image data, the B-mode image data generation unit 51 is not an essential component. In this case, the signal amplification unit 31 and the amplification correction unit 41 are also unnecessary.

The feature-data image data generation unit 52 generates the feature-data image data for displaying the information corresponding to the feature data in accordance with one of the plurality of display methods in accordance with a relationship between the feature data extracted by the feature-data extraction unit 43, and a threshold in the feature data, the threshold being constant regardless of a value of a display parameter that the image data has. The display method used here is selected by a display method selector 91 of the control unit 9 described later.

Information assigned to each pixel in the feature-data image data is determined depending on the data amount of the FFT data group when the frequency analysis unit 42 calculates the frequency spectrum. Specifically, for example, a pixel area corresponding to a data amount of one FFT data group is assigned with information corresponding to the feature data of the frequency spectrum calculated from the FFT data group. Note that, in the embodiment, although description is given such that the feature data used when generating the feature-data image data is only one type, the feature-data image data may be generated by using a plurality of types of feature data.

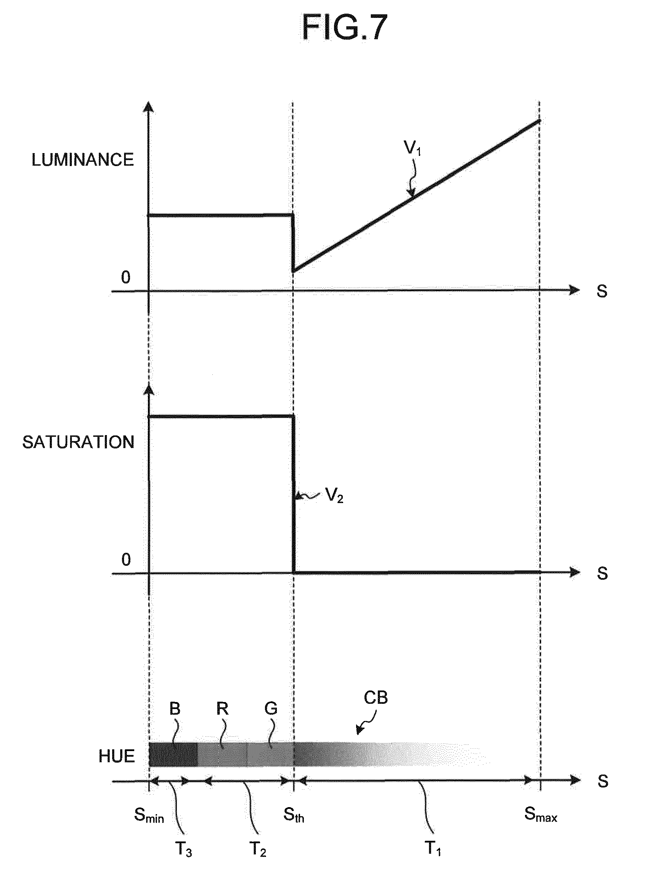

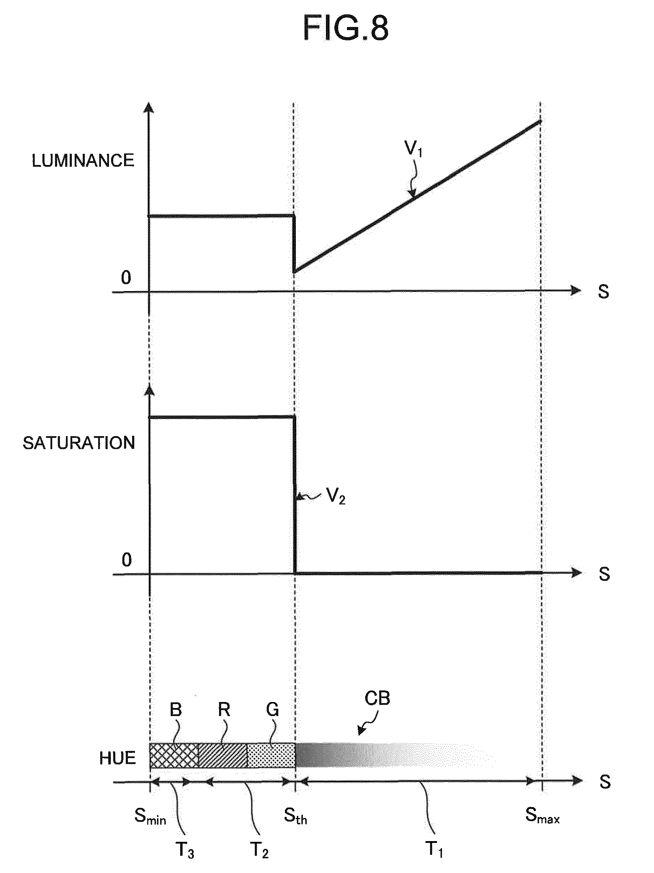

FIG. 7 is a diagram illustrating an example of a relationship between the feature data and the plurality of display methods when the feature-data image data generation unit 52 generates the feature-data image data. FIG. 8 is a diagram schematically illustrating the image illustrated in FIG. 7 in black and white. When illustrated in FIG. 7 and FIG. 8, information corresponding to the feature data has luminance, saturation, and hue as variables. The plurality of display methods determine specific values of these variables. When illustrated in FIG. 7 and FIG. 8, the feature-data image data generation unit 52 generates the feature-data image data when the feature data S is in a range of $S_{min} \le S \le S_{max}$. A threshold $S_{th}$ illustrated in FIG. 7 and FIG. 8 is a parameter required for imaging, such as gain and contrast, and is always constant without being affected by the display parameter that is variable during real-time observation. Such a threshold $S_{th}$ is determined depending on a type (substantially, a type of the ultrasonic probe 2 mounted in the scope) of the scope and a type of the specimen to be observed. The threshold $S_{th}$ is stored in a threshold information storage unit 84 (to be described later) that the storage unit 8 has, together with a relationship with the plurality of display methods illustrated in FIG. 7 and FIG. 8.

Examples of setting of luminance (curve $V_1$), saturation (curve $V_2$), and hue (color bar CB) in a magnitude relationship with the threshold $S_{th}$ will be described below with reference to FIG. 7 and FIG. 8. In FIG. 7 and FIG. 8, while gray scale display is employed so that the hue is constant when the feature data S is equal to or greater than the threshold $S_{th}$, color display is employed so that the hue varies when the feature data S is less than the threshold $S_{th}$. More specific description will be given below.

$$* \text{ When } S_{th} \leq S \leq S_{max}$$

The luminance increases as the feature data S increases. The saturation is zero regardless of the value of the feature data S, and the hue is constant regardless of the value of the feature data S (gray scale display). An area $T_1$ illustrated in FIG. 7 and FIG. 8 is an area where the value of the feature data S corresponds to a normal tissue.

$$* \text{ When } S_{min} \leq S < S_{th}$$

The luminance and saturation are constant regardless of the value of the feature data S. In addition, the hue changes sequentially from green G (illustrated by a dot pattern in FIG. 8), red R (illustrated by an obliquely striped pattern in FIG. 8), and blue B (illustrated by an oblique lattice pattern in FIG. 8) from the larger feature data S (color display). In FIG. 7 and FIG. 8, bandwidths of respective colors are equal. In addition, while an area $T_2$ (an area extending over green G and red R) illustrated in FIG. 7 and FIG. 8 is an area where the value of the feature data S corresponds to a lesion, an area $T_3$ (an area corresponding to blue B) is an area corresponding to a vascular channel. Note that, in this case, the luminance may be continuously decreased as the feature data S increases.

Meanwhile, the relationship between the feature data and the display methods illustrated in FIG. 7 and FIG. 8 is only one example. For example, it is preferable to set a number and type of colors, and the bandwidth of each color in color display in accordance with the relationship between the feature data S, and an organ to be observed and the scope to be used. In addition, regarding the type of colors, a user may be able to change settings via the input unit 6. Moreover, it is also possible to make settings to change hue in all the display areas and to switch the colors between both sides of the threshold. In addition, it is also possible to set a plurality of thresholds and to configure the display methods depending on a magnitude relationship with each threshold.

The storage unit 8 has an amplification factor information storage unit 81, a window function storage unit 82, a correction information storage unit 83, and a threshold information storage unit 84. The amplification factor information storage unit 81 stores a relationship (for example, the relationship illustrated in FIG. 2 and FIG. 3) between the amplification factor and the receiving depth as amplification factor information, the amplification factor being referred to when the signal amplification unit 31 performs amplification processing, and when the amplification correction unit 41 performs amplification correction processing. The window function storage unit 82 stores at least one window function among window functions, such as Hamming, Hanning, and Blackman. The correction information storage unit 83 stores information related to attenuation correction including Equation (1). The threshold information storage unit 84 stores the thresholds that are determined depending on a type (substantially, a type of the ultrasonic probe 2 mounted in the scope) of the scope and a type of the specimen to be observed. The threshold information storage unit 84 also associates each threshold with the plurality of display methods, and stores the thresholds and the display methods (see FIG. 7).

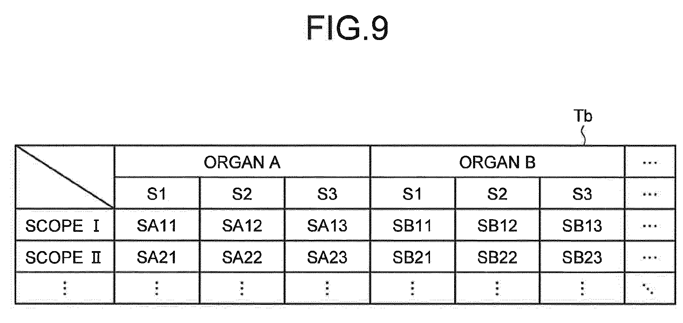

FIG. 9 is a diagram schematically illustrating an example of the thresholds stored in the threshold information storage unit 84. The table Tb illustrated in FIG. 9 records values of the thresholds according to the specimens to be observed and the types of scopes that each include the ultrasonic probe 2 for three pieces of feature data S1, S2, and S3. For example, when an organ A, which is a type of specimens, is to be observed by using a scope I, the thresholds of the feature data S1, S2, and S3 are SA11, SA12, and SA13, respectively. In addition, when an organ B is to be observed by using a scope II, the thresholds of the feature data S1, S2, and S3 are SB21, SB22, and SB23, respectively. It is preferable to set the thresholds as values that cancel variations in the feature data generated by a difference in performance of each scope. Specifically, for example, while a high threshold may be set for a scope that has a tendency to calculate high feature data, a low threshold may be set for a scope that has a tendency to calculate low feature data.

The storage unit 8 is implemented by using a read only memory (ROM) in which an operation program for the ultrasonic observation apparatus 1, a program that starts a specified operating system (OS), and the like are previously stored, and a random access memory (RAM) that stores computation parameters, data, and the like of each processing, and the like.

The control unit 9 has the display method selector 91 that selects the display method corresponding to the feature data extracted by the feature-data extraction unit 43 by referring to threshold information stored in the threshold information storage unit 84. The display method selector 91 outputs the selected display method to the feature-data image data generation unit 52.

The control unit 9 is implemented by using a central processing unit (CPU) that has computation and control functions. The control unit 9 exercises centralized control over the ultrasonic observation apparatus 1 by reading, from the storage unit 8, information and various programs including the operation program for the ultrasonic observation apparatus 1 which the storage unit 8 stores and contains, and by executing various types of arithmetic processing related to an operation method for the ultrasonic observation apparatus 1.

Note that it is also possible to record the operation program for the ultrasonic observation apparatus 1 in a computer-readable recording medium, such as a hard disk, a flash memory, a CD-ROM, a DVD-ROM, or a flexible disk, to allow wide distribution. Recording of various programs into the recording medium may be performed when a computer or the recording medium is shipped as a product, or may be performed through downloading via a communication network.

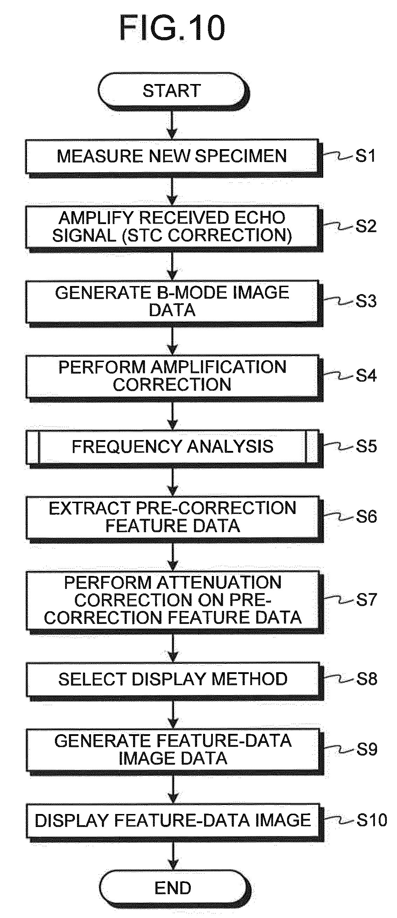

FIG. 10 is a flow chart illustrating an overview of the processing of the ultrasonic observation apparatus 1 that has the above configuration. Assume that the type of the ultrasonic probe 2 included in the ultrasonic observation apparatus 1 is previously recognized by the apparatus itself. For this purpose, for example, a connecting pin for allowing the processor to determine the type of scope (ultrasonic probe 2) may be provided at an end of the scope on a side of connection to the processor. This allows the processor side to determine the type of scope in accordance with a shape of the connected connecting pin of the scope. In addition, about the type of specimen to be observed, the user may previously input identification information by the input unit 6.

In FIG. 10, the ultrasonic observation apparatus 1 first performs measurement of a new specimen with the ultrasonic probe 2 (step S1).

Subsequently, the signal amplification unit 31 that receives the echo signal from the ultrasonic probe 2 performs amplification on the echo signal (step S2). Here, the signal amplification unit 31 performs amplification (STC correction) of the echo signal based on, for example, the relationship between the amplification factor and receiving depth illustrated in FIG. 2.

Subsequently, the B-mode image data generation unit 51 generates the B-mode image data by using the echo signal amplified by the signal amplification unit 31 (step S3). When the ultrasonic observation apparatus 1 is specialized for generation of the feature-data image data, this step S3 is unnecessary.

Subsequently, the amplification correction unit 41 performs amplification correction on a signal that is output from the transmitting and receiving unit 3 so that the amplification factor becomes constant regardless of the receiving depth (step S4). Here, the amplification correction unit 41 performs amplification correction based on, for example, the relationship between the amplification factor and the receiving depth illustrated in FIG. 3.

After step S4, the frequency analysis unit 42 calculates the frequency spectrum by performing frequency analysis through FFT computation (step S5).

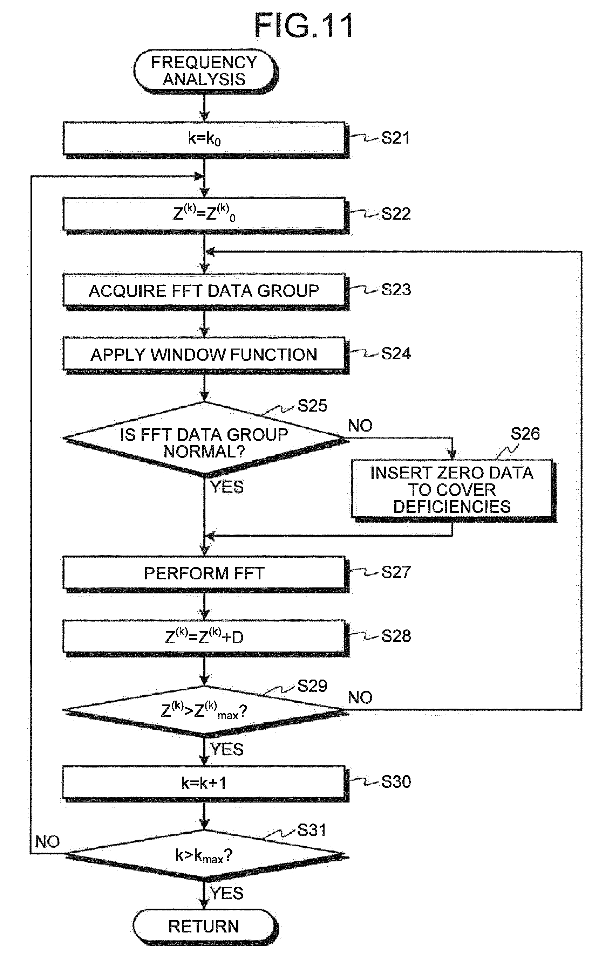

Here, processing (step S5) performed by the frequency analysis unit 42 will be described in detail with reference to a flow chart illustrated in FIG. 11. First, the frequency analysis unit 42 sets a counter k that identifies a sound ray to be analyzed as $k_0$ (step S21).

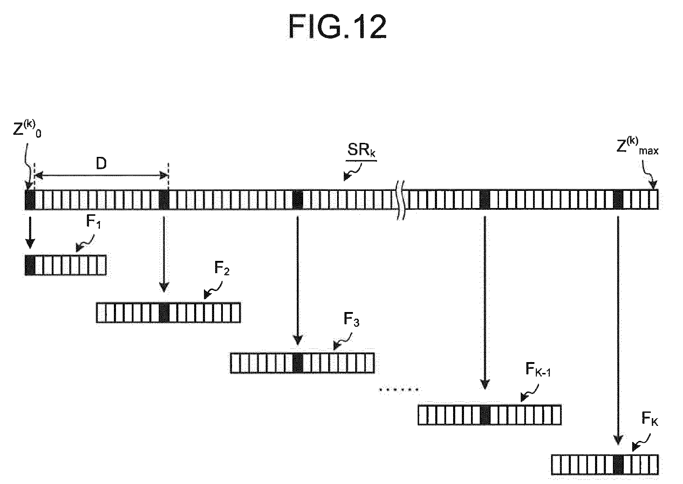

Subsequently, the frequency analysis unit 42 sets an initial value $Z^{(k)}_0$ of a data position (corresponding to the receiving depth) $Z^{(k)}$ that typifies a series of data groups (FFT data groups) acquired for FFT computation (step S22). FIG. 12 is a diagram schematically illustrating a data arrangement of one sound ray. In the sound ray $SR_k$ illustrated in FIG. 12, a white or black rectangle means one piece of data. The sound ray $SR_k$ is discretized at time intervals corresponding to a sampling frequency (for example, 50 MHz) in the analog-to-digital conversion performed by the transmitting and receiving unit 3. While FIG. 12 illustrates a case where a first data position of the sound ray $SR_k$ is set as the initial value $Z^{(k)}_0$, the position of the initial value can be set arbitrarily.

Subsequently, the frequency analysis unit 42 acquires the FFT data group at the data position $Z^{(k)}$ (step S23), and applies a window function stored in the window function storage unit 82 to the acquired FFT data group (step S24). By applying the window function to the FFT data group in this way, it is possible to avoid the FFT data group from being discontinuous at a border, and to prevent artifacts from occurring.

Subsequently, the frequency analysis unit 42 determines whether the FFT data group at the data position $Z^{(k)}$ is a normal data group (step S25). Here, the FFT data group needs to have a data number of a power of two. Hereinafter, assume that the data number of the FFT data group is $2^n$ (n is a positive integer). The FFT data group being normal means that the data position $Z^{(k)}$ is a $2^{n-1}$th position from the front in the FFT data group. In other words, the FFT data group being normal means that there are $2^{n-1} - 1$ (defined as N) pieces of data ahead of the data position $Z^{(k)}$, and that there are $2^{n-1}$ (defined as M) pieces of data behind the data position $Z^{(k)}$. In the case illustrated in FIG. 12, both the FFT data groups $F_2$ and $F_3$ are normal. Note that FIG. 12 illustrates a case of n = 4 (N = 7, M = 8) .

When the determination in step S25 results in that the FFT data group at the data position $Z^{(k)}$ is normal (step S25: Yes), the frequency analysis unit 42 proceeds to step S27 described later.

When the determination in step S25 results in that the FFT data group at the data position $Z^{(k)}$ is not normal (step S25: No), the frequency analysis unit 42 generates a normal FFT data group by inserting zero data to cover the deficiencies (step S26). The window function is applied to the FFT data group that has been determined not normal in step S25 before addition of zero data. Accordingly, even if zero data is inserted in the FFT data group, discontinuity of the data does not occur. After step S26, the frequency analysis unit 42 proceeds to step S27 described later.

In step S27, the frequency analysis unit 42 obtains the frequency spectrum made of complex numbers by performing FFT using the FFT data group (step S27). As a result, for example, the frequency spectrum curve $C_1$ as illustrated in FIG. 4 is obtained.

Subsequently, the frequency analysis unit 42 changes the data position $Z^{(k)}$ by a step width D (step S28). It is assumed that the step width D is previously stored in the storage unit 8. FIG. 12 illustrates a case of D = 15. It is desirable to match the step width D to a data step width used when the B-mode image data generation unit 51 generates the B-mode image data. However, when reduction of a computation amount in the frequency analysis unit 42 is desired, a value larger than the above data step width may be set.

Subsequently, the frequency analysis unit 42 determines whether the data position $Z^{(k)}$ is larger than a maximum value $Z^{(k)}_{max}$ in the sound ray $SR_k$ (step S29). When the data position $Z^{(k)}$ is larger than the maximum value $Z^{(k)}_{max}$ (step S29: Yes), the frequency analysis unit 42 increments the counter k by one (step S30). On the other hand, when the data position $Z^{(k)}$ is equal to or less than the maximum value $Z^{(k)}_{max}$ (step S29: No), the frequency analysis unit 42 returns to step S23. Thus, the frequency analysis unit 42 performs FFT on [{($Z^{(k)}_{max}$ - $Z^{(k)}_0$) /D} + 1] pieces of the FFT data group for the sound ray $SR_k$. Here, [X] represents a largest integer that does not exceed X.

After step S30, the frequency analysis unit 42 determines whether the counter k is larger than the maximum value $k_{max}$ (step S31). When the counter k is larger than $k_{max}$ (step S31: Yes), the frequency analysis unit 42 ends a series of steps of FFT processing. On the other hand, when the counter k is equal to or less than $k_{max}$ (step S31: No), the frequency analysis unit 42 returns to step S22.

Thus, the frequency analysis unit 42 performs multiple times of FFT computation on each of the ($k_{max}$ - $k_0$ + 1) sound rays.

Here, it is assumed that the frequency analysis unit 42 performs frequency analysis processing on all the areas in which the ultrasonic signal is received. However, the input unit 6 may receive setting input of a specific area of interest in advance to perform the frequency analysis processing only within the area of interest.

Following the frequency analysis processing of step S5 described above, the approximation unit 431 extracts pre-correction feature data by applying regression analysis to the frequency spectrum calculated by the frequency analysis unit 42 as approximation processing (step S6). Specifically, by calculating a linear expression that approximates intensity I (f, z) of the frequency spectrum in a frequency spectrum frequency band $f_L < f < f_H$ through regression analysis, the approximation unit 431 extracts the slope $a_0$, intercept $b_0$ (, and intensity $c_0$) that characterize this linear expression as pre-correction feature data. The straight line $L_{10}$ illustrated in FIG. 4 is an example of a regression line obtained by performing pre-correction feature-data extracting processing on the frequency spectrum curve $C_1$ in step S6.

Subsequently, the attenuation correction unit 432 performs attenuation correction processing on the pre-correction feature data extracted by the approximation unit 431 (step S7). For example, when the sampling frequency of data is 50 MHz, a time interval of data sampling is 20 (nsec). Here, when a speed of sound is assumed to be 1530 (m/sec), a distance interval of data sampling will be 1530 (m/sec) x 20 (nsec)/2 = 0.0153 (mm). Assuming that a data step number from first data of the sound ray to a data position of the FFT data group to be processed is n, the data position Z will be 0.0153nD (mm) by using the data step number n and the data step width D. The attenuation correction unit 432 calculates the slope a, intercept b (, and intensity c) which are feature data of the frequency spectrum, by substituting a value of the data position Z determined in this way into the receiving depth z of Equations (2) and (4) described above. Examples of a straight line corresponding to the feature data calculated in this way include the straight line $L_1$ illustrated in FIG. 5.

Steps S6 and S7 described above constitute a feature-data extraction step of extracting, when the feature-data extraction unit 43 approximates a frequency spectrum, at least one piece of feature data from the frequency spectrum.

Subsequently, the display method selector 91 selects the display method of information corresponding to the extracted feature data based on a value of the feature data extracted by the feature-data extraction unit 43, and on the threshold information stored in the threshold information storage unit 84. The display method selector 91 then outputs this selection result to the feature-data image data generation unit 52 (step S8).

Subsequently, the feature-data image data generation unit 52 generates the feature-data image data that displays information corresponding to the feature data in accordance with the display method selected by the display method selector 91, in accordance with the relationship between the feature data extracted in the feature-data extraction step (steps S6 and S7), and a threshold in the feature data, the threshold being constant regardless of the value of the display parameter that the image data has (step S9).

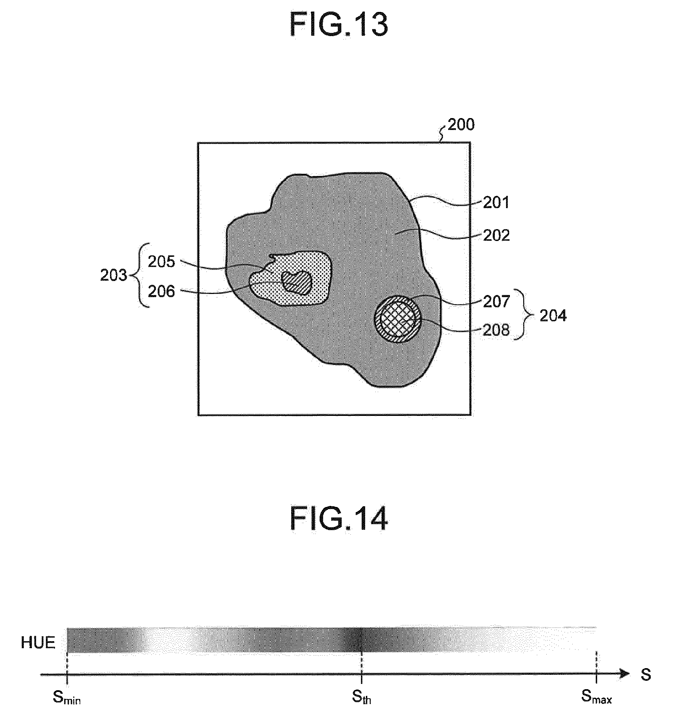

Subsequently, the display unit 7 displays the feature-data image generated by the feature-data image data generation unit 52 under control of the control unit 9 (step S10). FIG. 13 is a diagram illustrating an example of the feature-data image displayed by the display unit 7, and is a diagram illustrating a display example of the feature-data image generated based on the relationship between the feature data and the plurality of display methods illustrated in FIG. 7. A feature-data image 200 illustrated in FIG. 13 illustrates feature-data distribution in a specimen 201. The feature-data image 200 has one gray area 202 and two color areas 203 and 204 in the specimen 201. The color area 203 includes a closed green area 205 (illustrated in a dot pattern) and a red area 206 (illustrated in an obliquely striped pattern) that spreads inside the green area 205. The color area 204 includes an annular red area 207 (illustrated in an obliquely striped pattern) and a circular blue area 208 (illustrated in an oblique lattice pattern) that spreads inside the red area 207. It is considered that tissue characteristics differ in these areas, as is obvious from the colors of the areas. Specifically, a user who looks at the feature-data image 200 can determine that, as the tissue characteristics of the specimen 201, the gray area 202 is a normal tissue, the color area 203 is a lesion, and the color area 204 is a vascular channel, the feature-data image 200 being generated based on correspondence between the color and tissue characteristics described when FIG. 7 and FIG. 8 are described.

After step S10, the ultrasonic observation apparatus 1 ends a series of steps of processing. Here, the ultrasonic observation apparatus 1 may be adapted to repeat processing of steps S1 to S10 periodically.

According to the one embodiment of the present invention described above, the feature-data image data that displays information corresponding to the feature data in accordance with one of the plurality of display methods is generated, based on the relationship between the feature data extracted from the frequency spectrum received from the specimen to be observed and the threshold in the feature data, the threshold being constant regardless of the value of the display parameter that the image data has. Accordingly, it is possible to obtain the feature-data image data that has a strong relationship with a value of the feature data without being affected by the display parameter. Therefore, the embodiment allows the user to diagnose the tissue characteristics to be observed objectively and precisely based on the feature-data image.

In addition, according to the embodiment, the display method of information corresponding to the feature data extracted by the feature-data extraction unit is selected with reference to the threshold information storage unit that associates the value of the feature data including the threshold with the plurality of display methods and stores the value of the feature data and the plurality of display methods. In accordance with the selected display method, the feature-data image data generation unit generates the feature-data image data. Therefore, the relationship between the value of the feature data and the display method is absolute, enabling objective and highly precise diagnosis by the user even from this perspective.

In addition, according to the embodiment, the plurality of display methods are classified roughly into color display and gray scale display. Therefore, it is possible to generate the feature-data image data that clearly represents a difference in the tissue characteristics by, for example, applying the display method by color display to a point to highlight like a lesion among the tissue characteristics of a specimen, while applying the display method by gray scale display to a normal tissue.

In addition, according to the embodiment, it is possible to generate the feature-data image data that eliminates an influence of a hardware difference of the ultrasonic probe while taking the characteristics of the specimen into consideration, by determining the threshold in accordance with the specimen and the ultrasonic probe (or a type of scope in which the ultrasonic probe is mounted). As a result, the user is allowed to diagnose the tissue characteristics of the specimen with higher precision.

In addition, according to the embodiment, while the B-mode image data is generated based on a signal to which STC correction is applied for performing amplification with an amplification factor according to the receiving depth, the frequency spectrum is calculated after amplification correction is performed for offsetting only an influence of STC correction and making the amplification factor constant regardless of the receiving depth. After approximation processing is applied to this frequency spectrum, attenuation correction is performed on the pre-correction feature data obtained by the approximation processing. Thus, it is possible to correctly eliminate an influence of attenuation following propagation of an ultrasonic wave, and to prevent a decline in a frame rate of the image data generated based on the received ultrasonic wave. Therefore, according to the embodiment, it is possible to prevent the situation that the precision in the differentiation of the tissue characteristics of the specimen based on the frequency spectrum decreases due to the influence of attenuation.

(Other Embodiment)

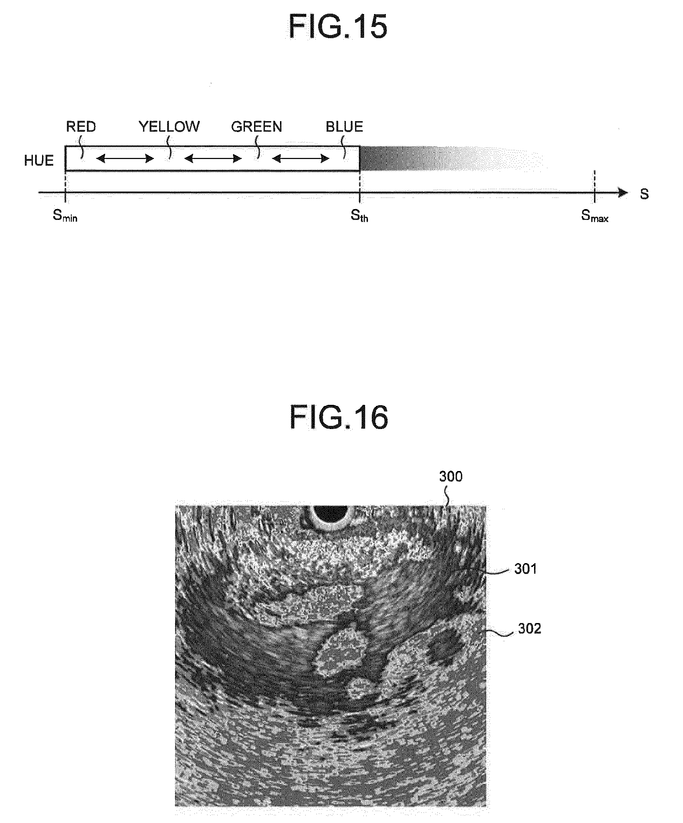

While the mode for carrying out the present invention has been described above, the present invention should not be limited only to one embodiment described above. For example, in the present invention, a setting method of hue when the feature-data image data generation unit 52 generates the feature-data image data is not limited to methods illustrated in FIG. 7 and FIG. 8. FIG. 14 is a diagram schematically illustrating another setting method of hue when the feature-data image data generation unit 52 generates feature-data image data. FIG. 15 is a diagram schematically illustrating the image illustrated in FIG. 14 in black and white. In cases illustrated in FIG. 14 and FIG. 15, hue is continuously varied in accordance with a change in a value of the feature data $S$ in $S_{min} \leq S < S_{th}$. Specifically, in FIG. 14 and FIG. 15, hue is continuously varied in order of from red to yellow to green to blue in accordance with variation of a wavelength as the feature data $S$ increases in a range of $S_{min} \leq S < S_{th}$. Here, a two-directional arrow in FIG. 15 schematically represents that hue varies continuously between the hues described at both ends of the arrow in accordance with the variation of the wavelength.

In addition, in the present invention, the plurality of display methods may be only two methods, color display and gray scale display. In this case, the display method selector 91 may select one of the two display methods when the feature data is equal to or greater than the threshold, and may select the other one of the two display methods when the feature data is less than the threshold.

Here, in FIG. 7 (and FIG. 8) and FIG. 14 (and FIG. 15), hue is provided in an area where the feature data is smaller than the threshold. This is because it is assumed that hue is provided to a tissue (for example, a lesion, such as a cancer) that is to be observed most when tissue characteristics are determined. Accordingly, depending on the type of specimen, an area where the feature data is larger than the threshold may be a lesion. Therefore, the area where hue is provided is not determined by a magnitude relationship with the threshold. It is preferable to have a configuration in which the area can be changed as appropriate in accordance with conditions, such as a type and characteristic of the specimen, and tissue characteristics that the user wants to examine.

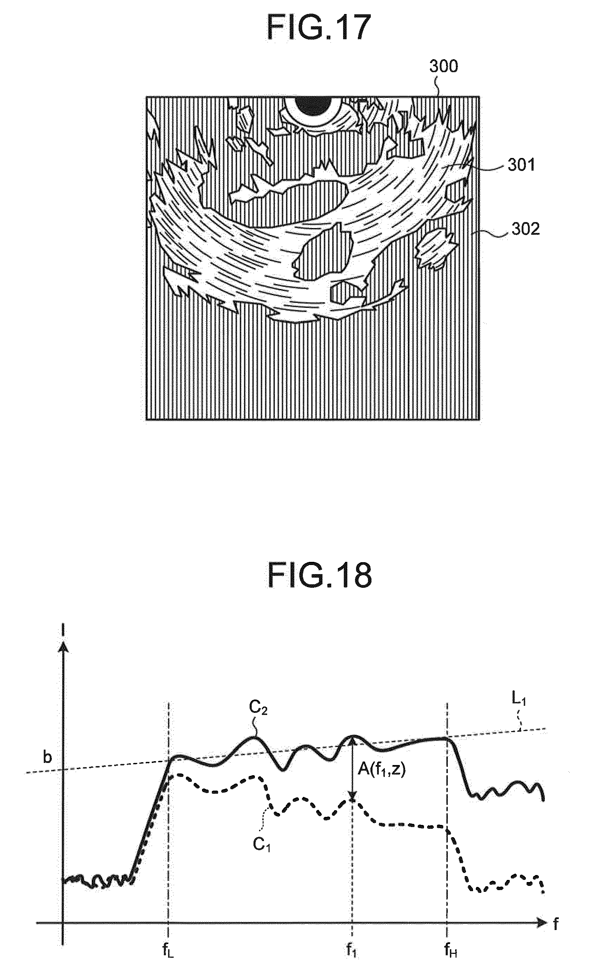

In addition, in the present invention, when displaying the feature-data image, the display unit 7 may display the B-mode image and the feature-data image side by side, and may superimpose the B-mode image on the feature-data image and display the feature-data image and the B-mode image. FIG. 16 is a diagram illustrating an example of a case where the display unit 7 superimposes the B-mode image on the feature-data image and displays the feature-data image and the B-mode image. FIG. 17 is a diagram schematically illustrating the image illustrated in FIG. 16 in black and white. A superimposed image 300 illustrated in FIG. 16 and FIG. 17 has a B-mode display area 301 in which the B-mode image is displayed as it is, and a superimposed display area 302 in which the feature-data image and the B-mode image are displayed in a superimposed state. Here, in FIG. 17, the variation of hue in the superimposed display area 302 is neglected, and the superimposed display area 302 is schematically illustrated in a vertically striped single pattern. In the superimposed image 300, it is assumed that a mixing ratio of the feature-data image and the B-mode image is previously set, but it is also possible to have a configuration in which the mixing ratio can be changed by input from the input unit 6. Thus, by displaying the feature-data image together with the B-mode image in the display unit 7, it becomes possible for the user, such as a doctor, to determine the tissue characteristics of the specimen together with information from the B-mode image, and to perform more precise diagnosis.

In addition, in the present invention, when the feature-data image that the display unit 7 is displaying is in a frozen state, the user may change the setting of the threshold arbitrarily.

In addition, in the present invention, after performing attenuation correction of the frequency spectrum, the feature-data extraction unit 43 may calculate the approximate expression of the after-correction frequency spectrum. FIG. 18 is a diagram schematically illustrating an overview of attenuation correction processing performed by the attenuation correction unit 432. As illustrated in FIG. 18, the attenuation correction unit 432 performs, on the frequency spectrum curve $C_1$, correction ($I(f, z) \to I(f, z) + A(f, z)$) for adding an attenuation amount $A(f, z)$ of Equation (1) to intensity $I(f, z)$ at each of all the frequencies $f$ ($f_L < f < f_H$) within a band. Thus, a new frequency spectrum curve $C_2$ is obtained with reduced contribution of attenuation following propagation of the ultrasonic wave. The approximation unit 431 extracts the feature data by applying regression analysis to the frequency spectrum curve $C_2$. The feature data extracted in this case is a slope a, intercept b (, and intensity c) of a straight line $L_1$ illustrated in FIG. 18. This straight line $L_1$ is identical to the straight line $L_1$ illustrated in FIG. 5.

In addition, in the present invention, the control unit 9 may collectively cause the amplification correction unit 41 to perform amplification correction processing, and cause the attenuation correction unit 432 to perform attenuation correction processing. This process is equivalent to changing definition of the attenuation amount of attenuation correction processing in step S7 of FIG. 10 as in the next Equation (6) without performing amplification correction processing in step S4 of FIG. 10.

$$A' = 2\alpha z f + \gamma(z) \qquad\qquad (6)$$

Here, y (z) of the right side is a difference in the amplification factors $\beta$ and $\beta_0$ at the receiving depth z, and is expressed as follows:

$$\gamma(z) = -\{(\beta_{th} - \beta_0)/z_{th}\}z + \beta_{th} - \beta_0 \quad (z \le z_{th}) \qquad (7)$$

$$\gamma(z) = 0 \quad (z > z_{th}) \qquad\qquad (8)$$

Thus, the present invention can include various embodiments without departing from technical ideas described in the claims.

Reference Signs List

| 1 | ultrasonic observation apparatus |
|---|---|
| 2 | ultrasonic probe |
| 3 | transmitting and receiving unit |
| 4 | computing unit |
| 5 | image processing unit |
| 6 | input unit |
| 7 | display unit |
| 8 | storage unit |
| 9 | control unit |
| 21 | signal converter |
| 31 | signal amplification unit |
| 41 | amplification correction unit |
| 42 | frequency analysis unit |
| 43 | feature-data extraction unit |
| 51 | B-mode image data generation unit |
| 52 | feature-data image data generation unit |
| 81 | amplification factor information storage unit |
| 82 | window function storage unit |
| 83 | correction information storage unit |
| 84 | threshold information storage unit |
| 91 | display method selector |
| 100 | B-mode image |
| 200 | feature-data image |
| 201 | specimen |
| 202 | gray area |
| 203, 204 | color area |
| 205 | green area |
| 206, 207 | red area |
| 208 | blue area |
| 300 | superimposed image |
| 301 | B-mode display area |
| 302 | superimposed display area |
| 431 | approximation unit |
| 432 | attenuation correction unit |

**Claims**

1. An ultrasonic observation apparatus comprising:

   an ultrasonic probe configured to transmit an ultrasonic signal to a specimen and to receive an ultrasonic wave reflected from the specimen;
   a frequency analysis unit configured to analyze a frequency of the ultrasonic wave received by the ultrasonic probe to calculate a frequency spectrum;
   a feature-data extraction unit configured to approximate the frequency spectrum calculated by the frequency analysis unit to extract at least one piece of feature data from the frequency spectrum; and
   a feature-data image data generation unit configured to generate feature-data image data for displaying information corresponding to the feature data in accordance with one of a plurality of display methods, depending on a relationship between the feature data extracted by the feature-data extraction unit and a threshold in the feature data, the threshold being constant regardless of a value of a display parameter of image data.

2. The ultrasonic observation apparatus according to claim 1, further comprising:

   a threshold information storage unit configured to associate a value of the feature data including the threshold with the plurality of display methods, and to store the value of the feature data including the threshold and the plurality of display methods; and
   a display method selector configured to select a display method of the information corresponding to the feature data extracted by the feature-data extraction unit from among the plurality of display methods stored in the threshold information storage unit, and to cause the feature-data image data generation unit to generate the feature-data image data in accordance with the selected display method.

3. The ultrasonic observation apparatus according to claim 2, wherein
the plurality of display methods are classified into color display in which hue varies depending on the value of the feature data, and gray scale display in which hue is constant regardless of the value of the feature data, and
the display method selector is configured to select one of the color display and the gray scale display in accordance with a magnitude relationship between the feature data and the threshold.

4. The ultrasonic observation apparatus according to any one of claims 1 to 3, wherein
the ultrasonic probe can be selected from a plurality of ultrasonic probes different in types from one another,
the threshold information storage unit stores the threshold according to types of the specimen and the ultrasonic probe, and
the feature-data image data generation unit is configured to generate the feature-data image data based on the threshold according to the types of the specimen and the ultrasonic probe.

5. The ultrasonic observation apparatus according to any one of claims 1 to 4, wherein the feature-data extraction unit comprises:

an approximation unit configured to perform approximation processing on the frequency spectrum calculated by the frequency analysis unit to calculate an approximate expression of the frequency spectrum; and
an attenuation correction unit configured to perform attenuation correction processing for reducing contribution of attenuation that occurs in accordance with a receiving depth and frequency of the ultrasonic wave, on the approximate expression calculated by the approximation unit to extract the feature data of the frequency spectrum.

6. The ultrasonic observation apparatus according to any one of claims 1 to 4, wherein the feature-data extraction unit comprises:

an attenuation correction unit configured to perform attenuation correction processing for reducing contribution of attenuation that occurs in accordance with a receiving depth and frequency of the ultrasonic wave, on the frequency spectrum calculated by the frequency analysis unit when the ultrasonic wave propagates; and
an approximation unit configured to perform approximation processing on the frequency spectrum corrected by the attenuation correction unit to extract the feature data of the frequency spectrum.

7. The ultrasonic observation apparatus according to claim 5 or 6, wherein the approximation unit is configured to approximate the frequency spectrum as an approximation target by a polynomial through regression analysis.

8. The ultrasonic observation apparatus according to claim 7, wherein the approximation unit is configured to approximate the frequency spectrum as the approximation target by a linear expression, and to extract, as the feature data, at least one of a slope of the linear expression, an intercept of the linear expression, and intensity that is defined by using the slope, the intercept, and a specific frequency included in a frequency band of the frequency spectrum.

9. A method for operating an ultrasonic observation apparatus that transmits an ultrasonic signal to a specimen and receives an ultrasonic wave reflected from the specimen, the method comprising:

a frequency analysis step of analyzing, by a frequency analysis unit, a frequency of the ultrasonic wave to calculate a frequency spectrum;
a feature-data extraction step of approximating, by a feature-data extraction unit, the frequency spectrum to extract at least one piece of feature data from the frequency spectrum; and
a feature-data image data generation step of generating, by a feature-data image data generation unit, feature-data image data for displaying information corresponding to the feature data in accordance with one of a plurality of display methods, depending on a relationship between the feature data extracted in the feature-data extraction step and a threshold in the feature data, the threshold being constant regardless of a value of a display parameter of image data.

10. A program for operating an ultrasonic observation apparatus that transmits an ultrasonic signal to a specimen and receives an ultrasonic wave reflected from the specimen, the program causing the ultrasonic observation apparatus to execute:

a frequency analysis step of analyzing, by a frequency analysis unit, a frequency of the ultrasonic wave to calculate a frequency spectrum;

a feature-data extraction step of approximating, by a feature-data extraction unit, the frequency spectrum to extract at least one piece of feature data from the frequency spectrum; and

a feature-data image data generation step of generating, by a feature-data image data generation unit, feature-data image data for displaying information corresponding to the feature data in accordance with one of a plurality of display methods, depending on a relationship between the feature data extracted in the feature-data extraction step and a threshold in the feature data, the threshold being constant regardless of a value of a display parameter of image data.

# FIG.1

EP 3 005 945 A1

# FIG.2

# FIG.3

# FIG.4

# FIG.5

# FIG.6

100

# FIG.7

# FIG.8

# FIG.9

Tb

| | ORGAN A | | | ORGAN B | | | ... |
|---|---|---|---|---|---|---|---|
| | S1 | S2 | S3 | S1 | S2 | S3 | ... |
| SCOPE I | SA11 | SA12 | SA13 | SB11 | SB12 | SB13 | ... |
| SCOPE II | SA21 | SA22 | SA23 | SB21 | SB22 | SB23 | ... |
| ⋮ | ⋮ | ⋮ | ⋮ | ⋮ | ⋮ | ⋮ | ⋱ |

# FIG.10

START

MEASURE NEW SPECIMEN ~S1

AMPLIFY RECEIVED ECHO
SIGNAL (STC CORRECTION) ~S2

GENERATE B-MODE IMAGE
DATA ~S3

PERFORM AMPLIFICATION
CORRECTION ~S4

FREQUENCY ANALYSIS ~S5

EXTRACT PRE-CORRECTION
FEATURE DATA ~S6

PERFORM ATTENUATION
CORRECTION ON PRE-
CORRECTION FEATURE DATA ~S7

SELECT DISPLAY METHOD ~S8

GENERATE FEATURE-DATA
IMAGE DATA ~S9

DISPLAY FEATURE-DATA IMAGE ~S10

END

# FIG.11

```
        ┌─────────────────────┐
        │     FREQUENCY        │
        │     ANALYSIS         │
        └─────────────────────┘
                  │
                  ▼
        ┌─────────────────────┐
        │      k=k₀           │ ～S21
        └─────────────────────┘
                  │
                  ▼
        ┌─────────────────────┐
        │   Z^(k)=Z^(k)_0     │ ～S22
        └─────────────────────┘
                  │
                  ▼
        ┌─────────────────────┐
        │ ACQUIRE FFT DATA GROUP│ ～S23
        └─────────────────────┘
                  │
                  ▼
        ┌─────────────────────┐
        │ APPLY WINDOW FUNCTION │ ～S24
        └─────────────────────┘
                  │
                  ▼
             S25
         ◇ IS FFT DATA GROUP ◇──NO──┐       S26
             NORMAL?                 ▼
                │YES        ┌─────────────────┐
                │           │ INSERT ZERO DATA │
                │           │    TO COVER      │
                │           │  DEFICIENCIES    │
                │           └─────────────────┘
                ▼◄───────────────────┘
        ┌─────────────────────┐
        │    PERFORM FFT       │ ～S27
        └─────────────────────┘
                  │
                  ▼
        ┌─────────────────────┐
        │   Z^(k)=Z^(k)+D     │ ～S28
        └─────────────────────┘
                  │
                  ▼
              S29
         ◇ Z^(k)>Z^(k)_max? ◇──NO──┐
                │YES
                ▼
        ┌─────────────────────┐
        │      k=k+1           │ ～S30
        └─────────────────────┘
                  │
                  ▼
              S31
    NO   ◇  k>k_max?  ◇
                │YES
                ▼
        ┌─────────────────────┐
        │      RETURN          │
        └─────────────────────┘
```

The flowchart steps:

- **S21**: $k=k_0$
- **S22**: $Z^{(k)}=Z^{(k)}_0$
- **S23**: ACQUIRE FFT DATA GROUP
- **S24**: APPLY WINDOW FUNCTION
- **S25**: IS FFT DATA GROUP NORMAL?
- **S26**: INSERT ZERO DATA TO COVER DEFICIENCIES
- **S27**: PERFORM FFT
- **S28**: $Z^{(k)}=Z^{(k)}+D$
- **S29**: $Z^{(k)}>Z^{(k)}_{max}$?
- **S30**: $k=k+1$
- **S31**: $k>k_{max}$?

# FIG.12

# FIG.13

# FIG.14

# FIG.15

HUE

RED   YELLOW   GREEN   BLUE

$S_{min}$   $S_{th}$   $S_{max}$

S

# FIG.16

300
301
302

# FIG.17

# FIG.18

**EP 3 005 945 A1**

<table>
<tr><td colspan="2"><b>INTERNATIONAL SEARCH REPORT</b></td><td>International application No.<br><br>PCT/JP2014/064559</td></tr>
</table>

<table>
<tr><td colspan="3">A. CLASSIFICATION OF SUBJECT MATTER<br><i>A61B8/00</i>(2006.01)i</td></tr>
<tr><td colspan="3">According to International Patent Classification (IPC) or to both national classification and IPC</td></tr>
</table>

B. FIELDS SEARCHED

Minimum documentation searched (classification system followed by classification symbols)
A61B8/00

Documentation searched other than minimum documentation to the extent that such documents are included in the fields searched
Jitsuyo Shinan Koho          1922–1996   Jitsuyo Shinan Toroku Koho   1996–2014
Kokai Jitsuyo Shinan Koho    1971–2014   Toroku Jitsuyo Shinan Koho   1994–2014

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br>A | WO 2012/63929 A1 (Olympus Medical Systems Corp.),<br>18 May 2012 (18.05.2012),<br>entire text; all drawings<br>& US 2013/30296 A1 & EP 2599441 A1<br>& JP 5307939 B2 | 1,2,5-10<br>3,4 |
| Y | WO 2006/106852 A1 (Hitachi Medical Corp.),<br>12 October 2006 (12.10.2006),<br>entire text; all drawings<br>& US 2009/149750 A1 & EP 1864612 A1<br>& JP 5334413 B2 | 1,2,5-10 |

☐ Further documents are listed in the continuation of Box C.    ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>13 June, 2014 (13.06.14) | Date of mailing of the international search report<br>24 June, 2014 (24.06.14) |
| Name and mailing address of the ISA/<br>Japanese Patent Office | Authorized officer |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012063975 A